# EUROPEAN PATENT SPECIFICATION

(11) **EP 3 352 930 B1**
(45) Date of publication and mention of the grant of the patent: **29.12.2021**
(21) Application number: 16849542.2
(22) Date of filing: 21.09.2016
(51) Int. Cl.: B22F 10/28, B22F 10/64, B33Y 70/00, B33Y 80/00, B33Y 10/00, B33Y 40/00, A61C 7/00, A61L 27/06, A61L 27/54, A61B 17/064, B22F 5/00, C22C 1/04, C22C 19/00, C22C 19/03, A61B 17/00, C22F 1/10, B22F 10/20, B33Y 50/02, B22F 3/24

(54) **SUPERELASTIC DEVICES MADE FROM NITIHF ALLOYS USING POWDER METALLURGICAL TECHNIQUES**
SUPERELASTISCHE VORRICHTUNGEN AUS NITIHF-LEGIERUNGEN MITTELS PULVERMETALLURGISCHER VERFAHREN
DISPOSITIFS SUPERÉLASTIQUES FABRIQUÉS À BASE D'ALLIAGES NITIHF AU MOYEN DE TECHNIQUES DE MÉTALLURGIE DES POUDRES

(30) Priority: 21.09.2015 US 201562221544 P
(43) Date of publication of application: 01.08.2018
(73) Proprietor: Confluent Medical Technologies, Inc., Fremont, CA 94539 (US)
(72) Inventor: DUERIG, Thomas, Fremont, CA 94539 (US); STEBNER, Aaron, Fremont, CA 94539 (US); NOEBE, Ronald D., Fremont, CA 94539 (US)
(74) Representative: J A Kemp LLP
(86) International application number: PCT/US2016/052960
(87) International publication number: WO 2017/053480

(56) References cited:
- EP-A1- 2 801 632
- EP-A2- 0 873 734
- WO-A1-2014/071135
- WO-A1-2014/071135
- WO-A1-2015/047128
- US-A1- 2013 209 262
- US-A1- 2013 328 975
- US-A1- 2015 004 042
- US-B2- 7 128 757
- US-B2- 7 128 757
- H. E. KARACA ET AL: "NiTiHf-based shape memory alloys", MATERIALS SCIENCE AND TECHNOLOGY, vol. 30, no. 13, 15 November 2014 (2014-11-15), pages 1530-1544, XP055564707, ISSN: 0267-0836, DOI: 10.1179/1743284714Y.0000000598

## Description

### BACKGROUND

Powder metallurgical techniques have been known for many years, including, more recently, several "so called" Additive Manufacturing (AM) methods, often colloquially referred to as "3-D printing." Perhaps the most advanced of these techniques as it relates to NiTi alloys ("Nitinol") is laser sintering. Laser sintering is a process by which prealloyed powders are spread on a surface in an even, very thin layer, then a laser is scanned over the powder to sinter individual particles to one another. Unsintered powder is then blown away, new powder spread, and the process repeated, building layer by layer, until a 3 dimensional structure is obtained. By controlling where the laser touches the particles and where it does not, complex three - dimensional shapes can be achieved, including shapes that cannot be produced by other means, such as a hollow sphere.

Another AM technique that could be used for Nitinol alloys feeds fine wire and selectively melts the wire in areas where the intended object is to be constructed. Melting can induced by lasers, arc welding, or a variety of other means.

One drawback of these methods, particularly as it pertains to Nitinol, is that the resulting material is weak, meaning that it has little resistance to convention deformation. This is important because efficient superelasticity requires that alloy resists conventional deformation in favor of deformation through phase transformation-conventional deformation compromised the superelastic properties, particularly the so-called residual set.

A useful measure of an alloy's resistance to conventional deformation is the Ultimate Tensile Strength, or "UTS." In metals in general, UTS can be increased by (i) solid solution strengthening such as in brass, (ii) by aging, such as in 17-4 PH stainless steel, or by (iii) cold working such as in copper. In conventional Nitinol, the first two mechanisms are not effective because one cannot add enough excess nickel to effective age or solid solution strengthen the alloy. Nitinol alloys are thus hardened through cold working. As stated above, this mechanism is not available to devices made through AM techniques since the very idea of AM is to produce the net shape. It is this problem that is addressed by this patent.

US 2015/004042 A1 discloses a method for manufacturing a three-dimensional biomedical device for fitting in a bone defect having an osteoinductive first area with a controlled porosity and a second area, which is produced by laser technology from an absorbent and from a first powder including one of ceramics, metals, metal alloys, bioactive glasses, lead zirconate titanate and biocompatible polymers, or mixtures thereof. The ratio of the porosities from the second area to the first area is equal or less than one, preferably from 0.001 to 0.9. The device is manufactured by laser technology including layering a powder onto a plate so that a layer of a predetermined thickness is formed; the laser beam selectively processes the powder to produce a processed layer, and, thus, layer after layer, the layers are joined together until the biomedical device is formed.

WO 2014/071135 A1 discloses a method and apparatus for fabricating an object. The method and apparatus entail applying a pulsed laser energy to a first quantity of a powder material on a substrate so as to fuse particles of the powder material into a first layer on the substrate, and then forming at least one additional layer on the first layer by applying a pulsed laser energy to at least a second quantity of the powder material on the first layer so as to fuse particles of the powder material into the at least one additional layer on the first layer. The pulsed laser energy is applied in a controlled manner such that solidification dynamics of the first and second quantities of the powder material are altered to promote at least one microstructural characteristic of the first and additional layer.

US 2004/249447 A1 discloses a radiopaque nitinol medical device such as a stent for use with or implantation in a body lumen.

EP 2 801 632 A1 discloses an endoprosthetic device including at least one structural member formed from a fatigue-resistant superelastic or shape-memory alloy. The fatigue-resistant superelastic or shape-memory alloy has an increased fatigue life that is superior to known nickel-titanium alloys. The minimum fatigue life may be due, at least in part, to the nickel-titanium alloy having at least one of an oxygen concentration of less than 50 ppm, a carbon concentration of less than 50 ppm. The superelastic or shape-memory nickel-titanium alloy of the core region comprises substantially only austenite.

H. E. KARACA ET AL, "NiTiHf-based shape memory alloys", MATERIALS SCIENCE AND TECHNOLOGY, (15 November 2014), vol. 30, no. 13, doi: 10.1179/1743284714Y.0000000598, ISSN 0267-0836, pages 1530 - 1544, XP055564707 discloses NiTiHf-based shape memory alloys.

### SUMMARY OF THE DISCLOSURE

In general, in one embodiment, a near net shape additive manufacturing method of fabricating a medical device for implantation in a human or animal body includes: (1) applying a suitable energy to a first quantity of a pre-alloyed metallic powder material comprising Titanium, Nickel and Hafnium on a substrate so as to fuse particles of the pre-alloyed powder material into a first layer on the substrate; (2) forming at least one additional layer on the first layer by applying the suitable energy to at least a second quantity of the pre-alloyed powder material on the first layer so as to fuse particles of the pre-alloyed powder material into the at least one additional layer on the first layer; and (3) repeating the applying and the forming steps to fabricate a near net shape medical device from the pre-alloyed powder material and wherein the pre-alloyed metallic powder material has a Hafnium atomic percentage of between 4-10%, Ni atomic percentage between 50.5 - 51.5 % with the remainder being Ti. The suitable energy source may be from one or a combination of laser sintering, selective laser sintering, directed light fabrication, laser engineered net shaping, and direct laser powder deposition.

This and other embodiments can include one or more of the following features. The controlled manner of applying the pulsed laser energy can cause the first and second quantities of the powder material to fully melt. The controlled manner of applying the pulsed laser energy can reduce at least one microstructural defect in the first layer and the at least one additional layer, and the at least one microstructural defect can be chosen from the group consisting of microcracks and porosity. The pre-alloyed metallic powder material including Nickel, Titanium and Hafnium can further include a filler material or an additive material. The near net shape medical device can be a component used in an orthopedic procedure to repair a joint. The component can be a pin, a nail, a screw or a staple. The component can be an intervertebral cage. The component can be a component used in an orthodontic procedure. The component can be a wire or a pin. The fabricated near net shape medical device can be subsequently aged such that the Af temperature is less than body temperature and the UTS is at least 900 MPa. The pre-alloyed metallic powder material can have a nickel content greater than 50 atomic percent. The fabricated near net shape medical device can have less than 2% residual set (plastic deformation) is observed after a 6% tensile deformation. The aging temperature can be between 350 and 550°C. The aging temperature can be between 400-600°C for 5 - 500 minutes. The aging process temperature and timing can be selected so that the UTS of the component increases by at least 100 MPa. The near net shape medical device can be fabricated for implantation into the human body. The fabricated near net shape medical device after performing the aging step can be aged such that the H-phase of the NiTiHf precipitate is present in the near net shape medical device.

In general, in one embodiment, a near net shape implantable medical device fabricated using an additive manufacturing technique using a pre-alloyed metallic powder material includes NiTHf, the implantable medical device having an Af temperature of less than body temperature and an UTS of at least 900 MPa.

This and other embodiments can include one or more of the following features. The near net shape medical device can be a component used in an orthopedic procedure to repair a joint. The component can be a pin, a nail, a screw or a staple. The near net shape medical device can be an intervertebral cage. The near net shape medical device can be a component used in an orthodontic procedure. The component can be a wire or a pin. A near net shape implantable device can have structure, shape or features to enhance bone or tissue in growth.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:

- FIG. 1: is a diagram of an exemplary additive manufacturing apparatus.
- FIG. 2: is an exemplary stress strain curve for super elastic materials.
- FIG. 3: is an exemplary flow chart of embodiments of the inventive methods.

### DETAILED DESCRIPTION

The present invention generally relates to methods and apparatuses adapted to perform additive manufacturing (AM) processes, and specifically, AM processes that employ energy beam to selectively fuse a metal alloy powder containing Hafnium material to produce an object. More particularly, the invention relates to methods and systems that use a pulsed, directed energy beam to achieve predetermined densification and microstructural evolution in AM processes use metal alloy powder comprising Nickel Titanium and Hafnium. In some embodiments, the near net shape NiTiHf component is fabricated with features and characteristics to enhance porous structure for bony in-growth and enhance fixation of implanted components.

Hafnium (Hf) additions to NiTi have been known for some time, researched because the addition of Hf can increase the transformation temperature of Nitinol when the Ti+Hf content exceeds 50 atomic percent. This property is useful for shape memory actuators. Because of the electronic similarity of Hf to Ti, a great deal of Hf can be added to the alloy without interfering with the martensitic transformation that is responsible for the shape memory effect-over 20 atomic percent. This is highly unusual-most ternary additions quickly suppress martensite making it impossible to add more that a couple percent. The Hf additions have also lead to a very fine precipitate that is effective in strengthening the alloy and resisting conventional deformation. This precipitate has been named the "H phase."

The invention herein is to use the H phase to harden AM components made from NiTiHf that are Ni-rich rather than Ti+Hf rich, and thus have transformation temperatures at or below body temperature, thereby providing efficient superelastic AM components.

It should be noted that while these methods are ideally applied to AM methods, the same benefit would be achieved with all near-net shape processing, including conventional powder metallurgical methods and even casting.

The term "AM processes" (also, "additive manufacturing" processes) as used herein refers to any process which results in a useful, three-dimensional object and includes a step of sequentially forming the shape of the object one layer at a time. AM processes include laser-net-shape manufacturing, direct metal laser sintering (DMLS), direct metal laser melting (DMLM), etc. A particular type of AM process uses a laser beam, to sinter or melt a powder material. AM processes often employ relatively expensive metal powder materials or wire as a raw material. An example of a 3DP process may be found in U.S. Pat. No. 6,036,777 to Sachs, issued Mar. 14, 2000. Additional details related to metal alloy additive manufacturing processes are provided in William E. Frazier. "Metal Additive Manufacturing: A Review." Journal of Materials Engineering and Performance 23 (6). (2014): 1917-1928 and J. W. Sears. Direct Laser Powder Deposition - "State of the Art." Schenectady, New York: Knolls Atomic Power Laboratory, Nov. 1999.

The present invention relates generally to AM processes as a rapid way to manufacture an object (article, component, part, product, etc.) where a multiplicity of thin unit layers are sequentially comprising NiTiHf to produce the object. More specifically, layers of a powder material are laid down and irradiated with suitable energy beam (e.g., laser beam) so that particles of the powder material within each layer are sequentially sintered (fused) or melted to solidify the layer. According to an aspect of the invention, a pulsed-laser additive manufacturing (AM) apparatus is employed to generate a pulsed laser beam and perform a laser melting method capable of producing a three-dimensional object by fully melting particles within successive layers of a powder material to form a solid homogeneous mass.

AM processes generally involve the buildup of one or more materials to make a net or near net shape (NNS) object, in contrast to subtractive manufacturing methods. Though "additive manufacturing" is an industry standard term (F2792 (withdrawn), superseded by ISO / ASTM 52900:2015), AM encompasses various manufacturing and prototyping techniques known under a variety of names, including freeform fabrication, 3D printing, rapid prototyping/tooling, etc. AM techniques are capable of fabricating complex components from a wide variety of materials. Generally, a freestanding object can be fabricated from a computer aided design (CAD) model. A particular type of AM process uses a laser beam, to sinter or melt a powder material, creating a solid three-dimensional object in which particles of the powder material are bonded together. Different material systems, for example, engineering plastics, thermoplastic elastomers, metals, and ceramics are in use. Laser sintering or melting is a notable AM process for rapid fabrication of functional prototypes and tools. Applications include patterns for investment casting, metal molds for injection molding and die casting, and molds and cores for sand casting. Fabrication of prototype objects to enhance communication and testing of concepts during the design cycle are other common usages of AM processes.

Laser sintering is a common industry term used to refer to producing three-dimensional (3D) objects by using a laser beam to sinter or melt a fine powder. More accurately, sintering entails fusing (agglomerating) particles of a powder at a temperature below the melting point of the powder material, whereas melting entails fully melting particles of a powder to form a solid homogeneous mass. The physical processes associated with laser sintering or laser melting include heat transfer to a powder material and then either sintering or melting the powder material. Although the laser sintering and melting processes can be applied to a broad range of powder materials, the scientific and technical aspects of the production route, for example, sintering or melting rate and the effects of processing parameters on the microstructural evolution during the layer manufacturing process have not been well understood. This method of fabrication is accompanied by multiple modes of heat, mass and momentum transfer, and chemical reactions that make the process very complex.

Laser sintering/melting techniques often entail projecting a laser beam onto a controlled amount of powder (usually a metal) material on a substrate, so as to form a layer of fused particles or molten material thereon. By moving the laser beam relative to the substrate along a predetermined path, often referred to as a scan pattern, the layer can be defined in two dimensions on the substrate, the width of the layer being determined by the diameter of the laser beam where it strikes the powder material. Scan patterns often comprise parallel scan lines, also referred to as scan vectors or hatch lines, and the distance between two adjacent scan lines is often referred to as hatch spacing, which is usually less than the diameter of the laser beam so as to achieve sufficient overlap to ensure complete sintering or melting of the powder material. Repeating the movement of the laser along all or part of a scan pattern enables further layers of material to be deposited and then sintered or melted, thereby fabricating a three-dimensional object.

Detailed descriptions of laser sintering/melting technology may be found in U.S. Pat. No. 4,863,538, U.S. Pat. No. 5,017,753, U.S. Pat. No. 5,076,869, U.S. Pat. No. 4,944,817, and U.S. Pat. Application Publication No. 2015/0273631. With this type of manufacturing process, a laser beam is used to selectively fuse a powder material by scanning cross-sections of the material in a bed. These cross-sections are scanned based on a three-dimensional description of the desired object. This description may be obtained from various sources such as, for example, a computer aided design (CAD) file, scan data, or some other source.

According to certain aspects of the invention, the powder material can be a metallic material, nonlimiting examples of which include, titanium and its alloys, nickel and its alloys, and Hafnium and its alloys. Methods of producing a three-dimensional structure may include depositing a first layer of one or more of the aforementioned powder materials on a substrate. At least one additional layer of powder material is deposited and then the laser scanning steps for each successive layer are repeated until a desired object is obtained. In fabricating a three-dimensional structure, the powder material can be either applied to a solid base or not. The article is formed in layer-wise fashion until completion. In the present invention, there is no particular limitation on the particle shape of the powder material used in an embodiment of the present invention. The average grain size of the powder material is, in an embodiment, about 10 to 100 µm.

In one embodiment, the AM process is carried out under an inert atmosphere. In another embodiment, the inert atmosphere is an atmosphere comprising a gas selected from the group consisting of helium, argon, hydrogen, oxygen, nitrogen, air, nitrous oxide, ammonia, carbon dioxide, and combinations thereof. In one embodiment, the inert atmosphere is an atmosphere comprising a gas selected from the group consisting of nitrogen (N₂), argon (Ar), helium (He) and mixtures thereof. In one embodiment, the inert atmosphere is substantially an argon gas atmosphere.

In another embodiment, the pulsed-laser AM apparatus comprises a build chamber within which an article can be fabricated, a movable build platform within the chamber and on which the article is fabricated, a powder material delivery system, and a laser delivery system. The powder material delivery system delivers a powder material to the build platform. In an optional embodiment, a heating system may be employed that is capable of heating the powder material and the platform with a heated gas. By conforming to the shape of the object, powder material is only needed for portions of the movable platform on which the process is to be performed.

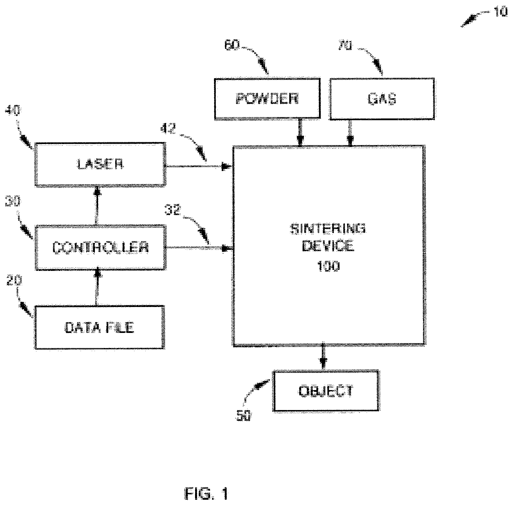

With reference now to FIG. 1, a diagram of a pulsed-laser AM apparatus 10 is depicted in accordance with one embodiment. In the particular example illustrated in FIG. 1, the apparatus 10 includes a pulsed-laser additive manufacturing (AM) device 100 there, in an embodiment, comprises a build chamber (not shown) within which an object 50 is to be fabricated and a movable build platform (not shown) within the build chamber and on which the object 50 is fabricated. The apparatus 10 further includes a pulsed-laser generating system 40 and a controller 30. In the illustrative example, a powder material 60 may be placed into the AM device 100 to create an object 50 using a pulsed laser beam 42 generated by the generating system 40. The object 50 may take various forms. The controller 30 may send control signals to the generating system 40 and control signals 32 to the AM device 100 to control the heating and, in some embodiments, melting of the powder material 60 to form the object 50. These control signals 32 may be generated using design data 20.

The pulsed laser beam 42 can be generated by pulsed excitation or by measures within the pulsed-laser generating system 40 (Q-switching or mode coupling). The pulsed laser beam 42 is not emitted continuously, in contrast with a continuous wave (CW) laser, but is emitted in a pulsed manner, i.e., in timely limited pulses.

In one embodiment, the generating system 40 is adapted to perform layer-by-layer and local fusing (melting or sintering) of the powder material 60. In one embodiment, the powder material 60 is an alloy sensitive to cracking in conventional laser sintering/melting processes, and the laser beam 42 is delivered in a controlled manner such that the solidification dynamics of the molten powder material 60 is altered to provide better microstructural characteristics of the resulting object 50. In one embodiment, the microstructural characteristics include one or more stress, strain and cracking states of the resolidified powder material 60. Without wishing to be limited to any particular theory, it is believed that the effect of pulse laser energy control on the material's solidification dynamics influences the temporal and spatial thermal gradients induced into the material by the energy deposition, the resulting transient, localized, temperature-dependent material properties commensurate with the thermal gradient, and the resulting material's physical response or microstructural characteristics.

According to some aspects of the invention, the laser beam 42 is applied in a pulsed manner utilizing laser welding parameters determined by the laser peak power, duty cycle of the pulse train, scan velocity (hatch speed), and hatch spacing (offset between adjacent scanned powder materials) to produce an article that is free or substantially free of microstructural defects, particularly microcracks and porosity.

The pulse frequency of the pulsed laser beam may be in a range of approximately 50 Hz to 50 KHz. In another embodiment, the pulse frequency is in the range of approximately 1 KHz to 50 KHz. In another embodiment, the pulse frequency is in the range of approximately 3 KHz to 50 KHz. In another embodiment, the pulse frequency is in the range of approximately 10 KHz to 50 KHz. In another embodiment, the pulse frequency is in the range of approximately 20 KHz to 50 KHz.

According to the present invention, the laser beam 42 can be modulated in a sinusoidal wave, rectangular wave, rectified sine wave, square wave, or any other waveform (e.g. sawtooth wave), which may be periodic or non-periodic or is repetitively shunted at a radio frequency. Such waves may have a ramp up, ramp down or both. In an embodiment, the degree of modulation can be optimized to meet the requirements for best performance of the solidification qualities.

Operator specified values can be computer fed into a waveform generator to specify appropriate time delay values and, in an embodiment, control the pulse energy of individual pulses that form into the burst pulse. Different profiles and repetition rates within the burst envelop with respect to the course or progress of the pulse peak intensity can therefore be arbitrarily defined and varied. For example, bursts of pulses can be generated where the pulse-energy envelope ramps up or ramps down monotonically or remains constant. Gaussian, Lorentzian, super-Gaussian, exponential rising, exponential falling and many other forms of pulse energy envelopes are anticipated by the invention. Combinations of short repetitive bursts, changes to the repetition rate, sinusoidal, and aperiodic distributions may be generated by the various embodiments described by the present invention. In certain embodiments, the modulation waveform is of high duty cycle (D=P_{avg}/P_{O}=*τ*f) to deliver sufficient pump energy without the risk of overdriving the laser.

In one embodiment, the laser scan velocity is in the range of from about 100 mm/s to about 2000 mm/s. In another embodiment, the laser scan velocity is in the range of from about 200 mm/s to about 1000 mm/s. In another embodiment, the laser scan velocity is in the range of from about 200 mm/s to about 400 mm/s. In yet another embodiment, lower scan velocities may be used, for example, in a range about 80 to about 400 mm/s.

In one embodiment, the hatch spacing is from about 0.02 mm to about 0.2 mm. In another embodiment, the hatch spacing is from about 0.04 mm to about 0.1 mm. In another embodiment, the hatch spacing is from about 0.05 mm to about 0.07 mm. Based on the hatch spacing and typical ranges for laser beam diameters, a typical beam overlap (b) may be about-1200% to about 50%.

In one embodiment, the duty cycle is from about 0.1 to about 0.95. In another embodiment, the duty cycle is from about 0.2 to about 0.8. In another embodiment, the duty cycle is from about 0.3 to about 0.7. In embodiments in which the powder material 60 is aluminum or an aluminum alloy, a particularly suitable duty cycle is believed to be about 0.5 to about 0.7. In other embodiments, a particularly suitable duty cycle is believed to be about 0.4 to about 0.6.

The thicknesses of a first layer and successive layers of the powder material 60 that are sequentially fused with the pulsed laser beam 42 are, in an embodiment, about 5 µm to about 2000 µm. In one embodiment, the powder material layer thickness scales with the available laser power. In another embodiment, the powder material layer thickness is about 10 µm to 200 µm. In another embodiment, the powder material layer thickness is about 20 µm-50 µm.

In one embodiment, the AM device 100 is capable of heating the powder material 60 with a heated gas 70 prior to the powder material 60 being subjected to the pulsed laser beam 42. Additionally, the heated gas 70 may heat other objects within the AM device 100 in a manner that may help maintain temperatures of already processed layers of the powder material 60 closer to the temperature of layers being fused.

The illustration of the apparatus 10 in FIG. 1 is not meant to imply physical and/or architectural limitations to the manner in which different environments may be implemented. For example, in other embodiments, the pulsed-laser generating system 40 may be implemented as part of the pulsed-laser AM device 100 rather than as a separate unit. The different units are illustrated as functional components, which may be combined or further separated into additional blocks depending on the particular implementation. In yet another example, the controller 30 may be implemented within the pulsed-laser AM device 100.

At first, the form and the material buildup of the object 50 are determined as design data 20 in a computer. The design data 20 also may take various forms. For example, the design data 20 may be a computer aided design (CAD) file or scan data. The CAD file of the three-dimensional electronic representation is typically converted into another file format known in the industry as stereolithographic or standard triangle language ("STL") file format or STL format. The STL format file is then processed by a suitable slicing program to produce an electronic file that converts the three-dimensional electronic representation of the object 50 into an STL format file comprising the object 50 represented as two-dimensional slices. Suitable programs for making these various electronic files are well-known to persons skilled in the art.

The layer information generated from this process is inputted into the controller 30, which produces the signals 32 delivered to a computer (not shown) of the AM device 100 to control the build platform thereof. The control signals 32 may also be utilized to control the supply of the powder material 60 and control the pulsed-laser generating system 40. The computer can also be used in particular as a control computer of the AM device 100. In the further course of the production of the object 50, the layer-by-layer buildup of the object 50 may take place in accordance with a, additive manufacturing method as previously described.

After a layer of the powder material 60 has been processed as a result of being melted by the pulsed laser beam 42, at least a portion of the build platform may be moved, for example, lowered within the build chamber. Thereafter, additional powder material 60 may be delivered to deposit another layer of the powder material 60 onto the previous layer and the build surface of the build platform. The additional layer of the powder material 60 can then be processed using the laser beam 42 delivered by the generating system 40. Each time a layer of the powder material 60 is deposited, a recoater may be sued to smooth the powder layer such that the powder layer defines a substantially planar surface. With this type of movement of the build platform, less powder material 60 may be used. Specifically, less powder material 60 is deposited onto areas in which movable stages have not moved downwards or have moved downwards less than other portions. The process repeats until near net shape article is completed.

Next, after the AM process, there is a method of treating the net shape NiTiHf article which is capable of transforming between martensitic and austenitic phases, to render the alloy pseudoelastic.

Alloys which are capable of transforming between martensitic and austenitic phases are generally able to exhibit a shape memory effect. The transformation between phases may be caused by a change in temperature: for example, a shape memory alloy in the martensitic phase will begin to transform to the austenitic phase when its temperature increases to a temperature greater than Aₛ, and the transformation will be complete when the temperature is greater than A_{f}. The reverse transformation will begin when the temperature of the alloy is decreased to a temperature less than Mₛ, and will be complete when the temperature is less than M_{f}. The temperatures Mₛ, M_{f}, Aₛ and A_{f} define the thermal transformation hysteresis loop of a shape memory alloy. Commonly known alloys which are capable of transforming in this way are based on nickel-titanium, for example as disclosed in U.S. Pat. No. 3,753,700, U.S. Pat. No. 4,505,767, U.S. Pat. No. 4,935,068 and U.S. Pat. No. 4,565,589, or on copper, for example as disclosed in U.S. Pat. No. 4,144,057 and U.S. Pat. No. 4,144,104.

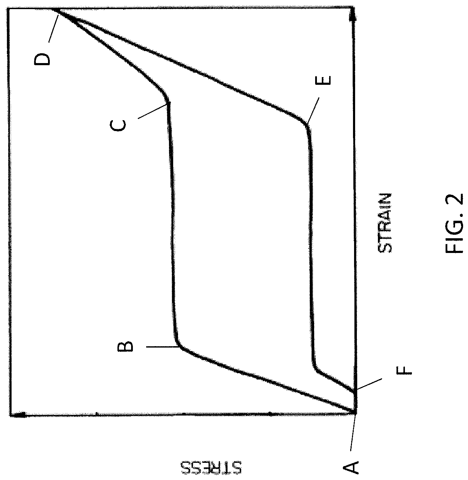

Fig. 2, which shows a representative stress-strain curve for an exemplary pseudoelastic NiTiHf alloy initially in an austenitic state and at a temperature above A_{f} but below M_{d}. At zero stress (point A), the alloy is in an austenitic state, assuming equilibrium conditions. As stress is applied, the austenite deforms elastically until point B, at which point sufficient stress is applied such that the austenite begins to transform to stress-induced martensite. Between points B and C, the transformation to martensite continues and the existing martensite is re-oriented to reflect the stress conditions. The transformation from austenite to stress-induced martensite is complete at or before point C. Between points C and D, the stress-induced martensite undergoes elastic deformation. If the alloy is released from its stress state when between points C and D, it should spring back (with some hysteresis effect) to point F along the reverse curve D-E-F to yield the so-called "pseudoelasticity" effect.

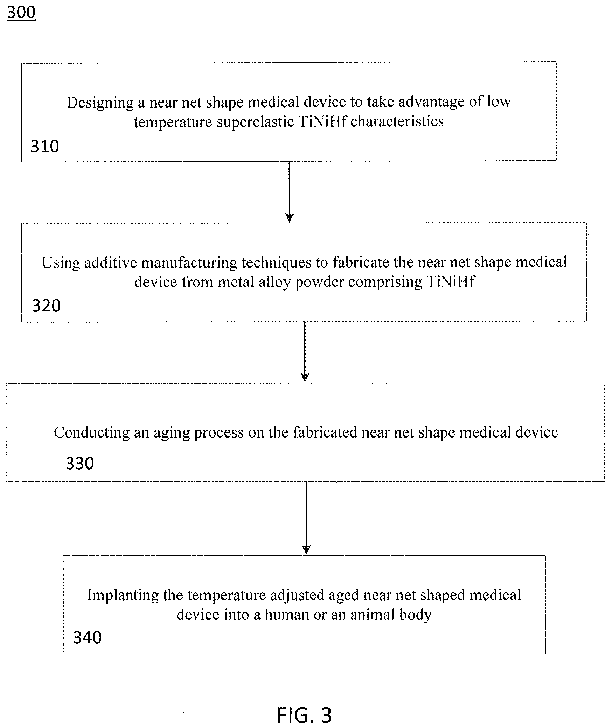

Fig. 3 is an exemplary flowchart of the steps to create a near net shape super elastic article containing nickel, titanium and hafnium.

First, at step 310, is the step of designing a near net shape medical device to take advantage of low temperature super elastic NiTiHf characteristics. A number of different articles may be designed to take advantage of the low temperature or near body temperature super elastic properties of the NiTiHf alloys described herein. Examples include components for orthopedic surgery such as, for example, rods, screws, staples and pins used in the repair of the joints of a human or mammal body. One exemplary device is described in U.S. Patent Application Publication No. 2016/0095638, titled "ORTHOPEDIC SCREW," and filed October 5, 2015. In some aspects, the additive manufacture design files includes voids, vias or other conduits and openings to permit or enable bony or tissue in growth on, in, or within the near net shape medical device. One exemplary osteogenic material in described in Assad, M et al. "INTERVERTEBRAL BODY FUSION USING A POROUS NITINOL ALLOY; 1-YEAR STUDY IN A SHEEP LUMBAR SPINAL MODEL." 49th Annual Meeting of the Orthopaedic Research Society. Still other medical device examples include the use of fixation and alignment plates. Other examples include interbody cages for spine fusion, inter-vertebral cages or other components for spinal orthopedical applications. Examples and additional details of illustrative embodiments are provided in U.S. Patent Application Publication No. 2004/0172130, titled "INTERVERTEBRAL CAGE," filed August 19, 2003, U.S. Patent No. 5,658,337, titled "INTERVERTEBRAL FUSION IMPLANT," U.S. Patent No. 5,162,327, titled "SURGICAL PROSTHETIC IMPLANT FOR VERTEBRAE," U.S. Patent No. 7,331,994, titled "INTERVERTEBRAL DISC REPLACEMENT PROSTHESIS," and U.S. Patent Application Publication No. 2011/0166600, titled "INTERSPINSOUS IMPLANTS AND METHODS," filed August 10, 2010. Other applications include wires and pins for orthodontic and endodontic systems and implants. Examples include U.S. Patent No. 5,876,434 titled "IMPLANTABLE MEDICAL DEVICES OF SHAPE MEMORY ALLOY," and U.S. Patent No. 4,490,112, titled "ORTHODONTIC SYSTEM AND METHOD." Appropriate electronic engineering files and other details are provided for use in the selected additive manufacturing system as described elsewhere herein.

Second, at step 320, is the step of using additive manufacturing techniques to fabricate the near net shape medical device from metal alloy powder comprising TiNiHf. The NiTiHf alloy includes a Ni atomic percentage that is greater than the sum of the summed atomic percentages of Ti + Hf. In the metal alloy powder there is 4-10 atomic percent Hf, 50.5 - 51.5 atomic percent Ni and the remainder is Ti.

Third, at step 330, is the step of conducting an aging process on the fabricated near net shape medical device. The aging process may take any of a wide variety of forms of exposing the fabricated medical device to high temperatures under one or more exposure times in the presence or absence of a gas, depending on particular embodiments. In one exemplary embodiment, a suitable aging process includes a heat treatment of over a time period sufficient to produces the desired ultimate tensile strength (UTS), crystalline structure or H phase precipitate. In one exemplary embodiment, the medical device is exposed to a temperature of 400-600°C for between 5 and 500 minutes. In one particular aspect, a specific aging treatment results in the UTS being increased by at least 100 MPa. In still another aspect, the subsequently aged near net shape medical device has properties such that the austenite final (A_{f}) temperature is less than body temperature and the UTS is at least 900 MPa. Body temperature is approximately 37° C or 98.6 ° F. In still another aspect, the aging process includes exposure of the near net shape medical device to an aging temperature is between 350 and 550°C. In still another aspect, after undergoing an aging process as described herein the near net shape medical device has characteristics of less than 2% residual set (plastic deformation) is observed after a 6% tensile deformation. In still another aspect, after the aging process there is near net shape medical devices formed from an NiTiHf alloy with at least 2% Hf that have been age hardened such that the H-phase is present within the NiTiHf structure. Additional variations and details of various aging and heat treating methods are described in International Patent Application Publication No. WO 99/42629, titled "PROCESS FOR THE IMPROVED DUCTILITY OF NITINOL."

Fourth, at step 340, is the step of implanting the temperature adjusted aged near net shape medical device into a human or animal body. As a result of the manufacturing and aging processes described herein the near net shape medical device now has appropriate characteristics for use in the body at low temperatures while still exhibiting the beneficial shape memory effect.

It must be noted that, as used in this specification and the appended claims, the singular forms "a," "an" and "the" include plural referents unless the content clearly dictates otherwise.

Unless defined otherwise, all technical and scientific terms used herein have the same meaning as commonly understood by one of ordinary skill in the art to which the invention pertains.

As will be appreciated by one having ordinary skill in the art, the methods and compositions of the invention substantially reduce or eliminate the disadvantages and drawbacks associated with prior art methods and compositions.

It should be noted that, when employed in the present disclosure, the terms "comprises," "comprising," and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

As required, detailed embodiments of the present invention are disclosed herein; however, it is to be understood that the disclosed embodiments are merely exemplary of the invention, which may be embodied in various forms. For example, instead of a single laser apparatus, two or more laser sources may be used. Therefore, specific structural and functional details disclosed herein are not to be interpreted as limiting, but merely as a basis for the claims and as a representative basis for teaching one skilled in the art to variously employ the present invention in virtually any appropriately detailed structure.

## Claims

1. A near net shape additive manufacturing method of fabricating a medical device for implantation in a human or animal body, the method comprising:
applying a pulsed laser energy to a first quantity of a pre-alloyed metallic powder material comprising Titanium, Nickel and Hafnium on a substrate so as to fuse particles of the pre-alloyed powder material into a first layer on the substrate;
forming at least one additional layer on the first layer by applying a pulsed laser energy to at least a second quantity of the pre-alloyed powder material on the first layer so as to fuse particles of the pre-alloyed powder material into the at least one additional layer on the first layer; and
repeating the applying and the forming steps to fabricate a near net shape medical device from the pre-alloyed powder material; and
wherein the pre-alloyed metallic powder material has:
a Hafnium atomic percentage of between 4-10%, Ni atomic percentage between 50.5 - 51.5 % with the remainder being Ti.

2. The additive manufacturing method of claim 1, wherein the controlled manner of applying the pulsed laser energy causes the first and second quantities of the powder material to fully melt.

3. The additive manufacturing method of claim 1, wherein the near net shape medical device is a component used in an orthopedic procedure to repair a joint, and optionally wherein the component is a pin, a nail, a screw or a staple.

4. The additive manufacturing method of claim 1 wherein the component is an intervertebral cage.

5. The additive manufacturing method of claim 1 wherein the component is a component used in an orthodontic procedure, and optionally wherein the component is a wire or a pin.

6. The additive manufacturing method of claim 1, wherein the fabricated near net shape medical device is subsequently aged between 350 and 550°C.

7. The additive manufacturing method of claim 1:
wherein the fabricated near net shape medical device is subsequently aged between 400-600°C for 5 - 500 minutes.

8. The additive manufacturing method of claim 1, wherein the near net shape medical device is fabricated for implantation into the human body.

9. The additive manufacturing method of claim 1 wherein the fabricated near net shape medical device is subsequently aged and wherein the fabricated near net shape medical device after performing the aging step is aged such that the H-phase of the NiTiHf precipitate is present in the near net shape medical device.

## Patentansprüche

1. Additives, endformnahes Fertigungsverfahren zum Fertigen einer medizinischen Vorrichtung für die Implantation in einen menschlichen oder tierischen Körper, wobei das Verfahren umfasst:
Anwenden einer gepulsten Laserenergie auf eine erste Menge eines vorlegierten metallischen Pulvermaterials, umfassend Titan, Nickel und Hafnium, auf einem Substrat, um Partikel des vorlegierten Pulvermaterials zu einer ersten Schicht auf dem Substrat zu verschmelzen;
Ausbilden wenigstens einer zusätzlichen Schicht auf der ersten Schicht durch Anwenden einer gepulsten Laserenergie auf wenigstens eine zweite Menge des vorlegierten Pulvermaterials auf der ersten Schicht, um Partikel des vorlegierten Pulvermaterials in die wenigstens eine zusätzliche Schicht auf der ersten Schicht zu verschmelzen; und
Wiederholen der Anwendungs- und der Ausbildungsschritts, um eine endformnahe medizinische Vorrichtung aus dem vorlegierten Pulvermaterial zu fertigen; und
wobei das vorlegierte metallische Pulvermaterial aufweist:
einen Hafnium-Atomprozentsatz von zwischen 4-10 %, einen Ni-Atomprozentsatz zwischen 50,5-51,5 %, wobei der Rest Ti ist.

2. Additives Fertigungsverfahren nach Anspruch 1, wobei die kontrollierte Art und Weise des Anwendens von gepulster Laserenergie bewirkt, dass die erste und zweite Menge des Pulvermaterials vollständig schmelzen.

3. Additives Fertigungsverfahren nach Anspruch 1, wobei die endformnahe medizinische Vorrichtung eine Komponente ist, die in einem orthopädischen Verfahren für die Reparatur eines Gelenks verwendet wird, und optional wobei die Komponente ein Stift, ein Nagel, eine Schraube oder eine Klammer ist.

4. Additive Fertigungsverfahren nach Anspruch 1, wobei die Komponente ein Zwischenwirbelkäfig ist.

5. Additives Fertigungsverfahren nach Anspruch 1, wobei die Komponente eine Komponente ist, die für ein kieferorthopädisches Verfahren verwendet wird, optional wobei die Komponente ein Draht oder ein Stift ist.

6. Additives Fertigungsverfahren nach Anspruch 1, wobei die hergestellte endformnahe medizinische Vorrichtung nachfolgend zwischen 350 und 550 °C ausgehärtet wird.

7. Additives Fertigungsverfahren nach Anspruch 1:
wobei die hergestellte endformnahe medizinische Vorrichtung nachfolgend zwischen 400-600 °C für 5-500 Minuten ausgehärtet wird.

8. Additives Fertigungsverfahren nach Anspruch 1, wobei die endformnahe medizinische Vorrichtung für eine Implantation in den menschlichen Körper hergestellt wird.

9. Additives Fertigungsverfahren nach Anspruch 1, wobei die hergestellt endformnahe medizinische Vorrichtung nachfolgend ausgehärtet wird und wobei die hergestellt endformnahe medizinische Vorrichtung nach dem Durchführen des Aushärteschritts derart ausgehärtet wird, dass die H-Phase des NiTiHf-Niederschlags in der endformnahen medizinischen Vorrichtung vorliegt.

## Revendications

1. Procédé de fabrication additive de forme quasiment nette pour la fabrication d'un dispositif médical destiné à être implanté dans un corps humain ou animal, le procédé comprenant :
l'application d'une énergie laser pulsée à une première quantité d'un matériau en poudre métallique pré-allié comprenant du titane, du nickel et de l'hafnium sur un substrat de manière à fusionner des particules du matériau en poudre pré-allié en une première couche sur le substrat ;
la formation d'au moins une couche supplémentaire sur la première couche en appliquant une énergie laser pulsée à au moins une seconde quantité du matériau en poudre pré-allié sur la première couche de manière à fusionner des particules du matériau en poudre pré-allié dans l'au moins une couche supplémentaire sur la première couche ; et
la répétition des étapes d'application et de formage pour fabriquer un dispositif médical de forme quasiment nette à partir du matériau en poudre pré-allié ; et
dans lequel le matériau en poudre métallique pré-allié a :
un pourcentage atomique de Hafnium compris entre 4 et 10%, un pourcentage atomique de Ni compris entre 50,5 et 51,5%, le reste étant du Ti.

2. Procédé de fabrication additive selon la revendication 1, dans lequel la manière contrôlée d'appliquer l'énergie laser pulsée fait fondre complètement les première et deuxième quantités du matériau en poudre.

3. Procédé de fabrication additive selon la revendication 1, dans lequel le dispositif médical de forme quasiment nette est un composant utilisé dans une procédure orthopédique pour réparer une articulation, et éventuellement dans lequel le composant est une broche, un clou, une vis ou une agrafe.

4. Procédé de fabrication additive selon la revendication 1 dans lequel le composant est une cage intervertébrale.

5. Procédé de fabrication additive selon la revendication 1, dans lequel le composant est un composant utilisé dans une procédure orthodontique, et éventuellement dans lequel le composant est un fil ou une broche.

6. Procédé de fabrication additive selon la revendication 1, dans lequel le dispositif médical fabriqué de forme quasiment nette est ensuite vieilli entre 350 et 550°C.

7. Procédé de fabrication additive selon la revendication 1 :
dans lequel le dispositif médical fabriqué de forme quasiment nette est ensuite vieilli entre 400 et 600°C pendant 5 à 500 minutes.

8. Procédé de fabrication additive selon la revendication 1, dans lequel le dispositif médical de forme quasiment nette est fabriqué pour être implanté dans le corps humain.

9. Procédé de fabrication additive selon la revendication 1, dans lequel le dispositif médical de forme quasiment nette fabriqué est ensuite vieilli et dans lequel le dispositif médical fabriqué de forme quasiment nette après avoir effectué l'étape de vieillissement est vieilli de telle sorte que la phase H du précipité de NiTiHf est présente dans le dispositif médical de forme quasiment nette.
